# EUROPÄISCHE PATENTSCHRIFT

(11) **EP 1 018 318 B1**
(45) Veröffentlichungstag und Bekanntmachung des Hinweises auf die Patenterteilung: **23.04.2014**
(21) Anmeldenummer: 99811121.5
(22) Anmeldetag: 08.12.1999
(51) Int. Cl.: A61B 17/72

(54) **Marknagel**
Intramedullary nail
Clou intramédullaire

(30) Priorität: 07.01.1999 EP 99810010
(43) Veröffentlichungstag der Anmeldung: 12.07.2000
(73) Patentinhaber: Zimmer GmbH, 8404 Winterthur (CH)
(72) Erfinder: Weigum, Hans, 4435 Niederdorf (CH)
(74) Vertreter: Manitz, Finsterwald & Partner GbR

(56) Entgegenhaltungen:
- EP-A- 0 099 642
- DE-A- 2 657 303
- DE-C- 767 879
- US-A- 2 490 364

## Beschreibung

Die Erfindung betrifft einen Marknagel, insbesondere einen Femurmarknagel für Kinder, mit den Merkmalen des Anspruchs 1.

Zur Frakturheilung bei gebrochenen Oberschenkelknochen werden Femurmarknägel verwendet. Bei bekannten Marknägeln, die steif sind, wird der Insertionspunkt so gewählt, dass dieser auf die proximale Verlängerung der Markhöhle zu liegen kommt. Bei Kindern hätte eine Knochenöffnung an diesem Insertionspunkt eine schädigende Wirkung auf die Blutversorgung am Schenkelhals und auf das Knochenwachstum zur Folge. Daher rät die Fachwelt von der Verwendung von steifen Femurmarknägeln bei Kindern ab.

Ein zur Behandlung von Knochenfrakturen dienender Stift, der an seinem distalen Ende aufspreizbar ist, ist aus US 2,490,364 bekannt.

Eine Längselastische für Innenschiene für Röhrenknochen ist aus DE767879 bekannt. EP 99642 A2 beschreibt einen elastischer Stift für Röhrenknochen.

Es ist Aufgabe der Erfindung, Mittel zu schaffen, mittels derer die genannte Frakturheilung auch bei Kindern unterstützt werden kann. Diese Aufgabe wird durch den in Anspruch 1 definierten Marknagel gelöst.

Der Marknagel, insbesondere ein Femurmarknagel für Kinder, umfasst ein Rohr und ein in das Rohr einschiebbares Element oder mehrere solche Elemente. Das Rohr ist ein elastischer Zylinder, der sich zwischen einem Mündungsbereich am proximalen Ende und einem Spitzenbereich am distalen Ende erstreckt. Mittels schlitzförmiger, in Rohrrichtung weisender Durchbrüche ist das Rohr biegeweich ausgebildet. Die eingeschobenen Elemente bilden zumindest lokale Verstärkungen, durch die das Rohr eine über seine Länge global wirkende Versteifung erhält.

Bei einem chirurgischen Verfahren zur Fixierung eines gebrochenen Oberschenkelknochens mit einem erfindungsgemässen Marknagel wird dieser seitlich am Knochen zugeführt. Der Insertionspunkt wird dorsal, lateral von der Crista intertrochanterica gewählt. Über der Eintrittsstelle am Knochen liegt der M. quadratus femoris. Zur Einführung des Marknagels wird die Markhöhle schräg von oben aufgebohrt, und es wird beispielsweise mit einem flexiblen Bohrer ein Kanal hergestellt. Das Rohr des Marknagels - ohne Verstärkungselemente - kann dann unter Verwendung eines Gleithammers eingetrieben werden. Dank seines biegeweichen Bereichs ist das Rohr so elastisch, dass es sich am gewählten Insertionspunkt im erforderlichen Mass krümmen lässt. Nachdem das Rohr eingeführt ist, werden die Verstärkungselemente beispielsweise mittels eines Montagestabes eingefügt. Dabei wird die Biegesteifigkeit des Marknagels so vergrössert, dass die für die Frakturheilung erforderliche Fixation sichergestellt ist. Mit den Verstärkungselementen ergibt sich auch eine radiale Verankerung des Rohrs in der Markhöhle, nämlich aufgrund einer Erweiterung des Querschnitts an den Stellen, wo er sich zuvor beim Einführen des Rohrs infolge von Krümmungen und Verengungen der Markhöhle durch Deformierungen verengt hat.

Die abhängigen Ansprüche 2 bis 12 beziehen sich auf vorteilhafte Ausführungsformen des erfindungsgemässen Marknagels.

Nachfolgend wird die Erfindung anhand der Zeichnungen erläutert. Es zeigen:
- Fig. 1: einen linken Oberschenkelknochen - von innen, medial, und 90° um seine Achse gedreht, von hinten, dorsal - mit einem Insertionspunkt für den erfindungsgemässen Marknagel,
- Fig. 2: eine Seitenansicht eines erfindungsgemässen Marknagels,
- Fig. 3: einen Montagestab mit drei Verstärkungselementen,
- Fig. 4: ein Verstärkungselement,
- Fig. 5: eine zweite Ausführungsform des erfindungsgemässen Marknagels,
- Fig. 6: ein Detail zum Marknagel der Fig. 5,
- Fig. 7: einen Querschnitt durch einen versteiften Marknagel,
- Fig. 8: einen Versteifungskörper,
- Fig. 9: ein Detail zum Marknagel der Fig. 1,
- Fig. 10: eine dritte Ausführungsform des erfindungsgemässen Marknagels,
- Fig. 11: einen Querschnitt durch den Marknagel der Fig. 10,
- Fig. 12: ein Schrägbild zu einem eingefügten Verstärkungselement,
- Fig. 13: eine vierte Ausführungsform des erfindungsgemässen Marknagels,
- Fig. 14: einen Querschnitt durch den in Fig. 13 gezeigten Marknagel mit Verstärkungselement,
- Fig. 15: ein Mündungsstück zu einer weiteren Ausführungsform des erfindungsgemässen Marknagels und
- Fig. 16, 17: einen erfindungsgemässen Marknagel mit mehreren rohrfömig angeordneten Stäben.

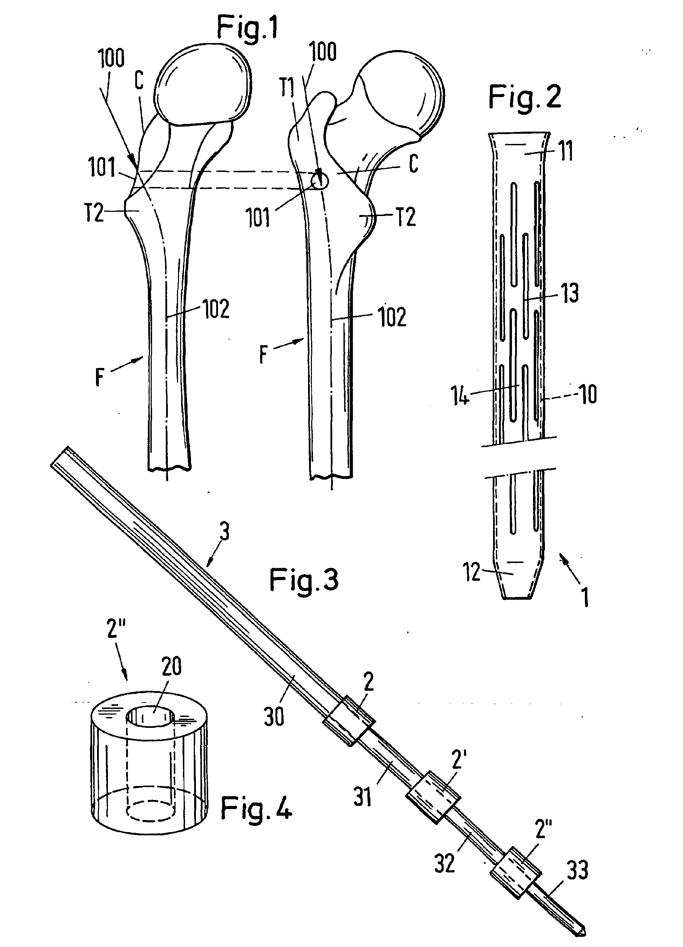

Es ist in Fig. 1 anhand zweier Ansichten der obere Teil eines Oberschenkelknochens F dargestellt, mit dem Trochanter major T1, dem Trochanter minor T2 und der Crista intertrochanterica C. Mit dem Pfeil 100 ist gezeigt, an welcher Stelle 101 und in welcher Richtung der erfindungsgemässe Marknagel 1 (Fig. 2) in die Markhöhle eingeführt werden soll. Strichpunktiert ist die Mittellinie 102 des Kanals eingezeichnet, in den der Marknagel 1 zu liegen kommt. Wie bei der Innenansicht, links, ersichtlich ist, muss aus geometrischen Gründen der Marknagel 1 an der Insertionsstelle 101 beim Einführen gekrümmt werden. In der Markhöhle muss er anschliessend wieder gestreckt oder leicht gebogen sein.

Der Marknagel 1 - siehe Fig. 2 - umfasst ein Rohr 10 und ein in das Rohr 10 einschiebbares Element oder mehrere solche Elemente (nicht sichtbar). Das Rohr 10 ist ein dünnwandiger, elastischer Zylinder, der sich zwischen einem Mündungsbereich 11 am proximalen Ende und einem Spitzenbereich 12 am distalen Ende erstreckt und der mittels schlitzförmiger, in Rohrrichtung weisender Durchbrüche 13 biegeweich ausgebildet ist. Das Material 14 des Zylinders 10 hat im wesentlichen die Form von Stangen, die parallel zur Zylinderachse angeordnet sind.

Die genannten Elemente bilden nach dem Einschieben lokale Verstärkungen, die in radialer Richtung steif sind. Fig. 3 zeigt einen Montagestab 3 mit aufgesteckten Verstärkungskörpem 2, 2' und 2", welche solche einschiebbaren Elemente sind. Sie haben axiale Bohrungen 20 mit verschiedenen Durchmessern. Der Montagestab 3 hat eine entsprechende Abstufung in Segmente 30, 31, 32 und 33 mit verschiedenen Durchmessern. Das Schrägbild der Fig. 4 zeigt ein einzelnes der einschiebbaren Elemente, nämlich den Verstärkungskörper 2" mit dem kleinsten Bohrungsdurchmesser. Die Verstärkungskörper 2, 2' und 2" werden mit dem Montagestab 3 in das Rohr 10 eingebracht, wobei sie beispielsweise einzeln unter Verwendung eines Gleithammers eingeschoben werden. Die eingeschobenen Elemente 2, 2' und 2" bilden lokale Verstärkungen, durch die das Rohr 10 eine über seine Länge global wirkende Versteifung erhält. Die Elemente 2, 2' und 2" haben an den verstärkten Stellen jeweils einen festen Sitz, der sich aufgrund von Reibkräften zwischen den sich berührenden Oberflächen ergibt.

Das Rohr 10 ist metallisch; es besteht beispielsweise aus einem rostfreien Stahl. Die Verstärkungselemente 2 sind mit Vorteil aus einem Kunststoff, beispielsweise aus Polyacetal POM, hergestellt, oder auch aus einem Gemisch von Polymeren, die zumindest zu einem Teil bioresobierbare Polymere sein können.

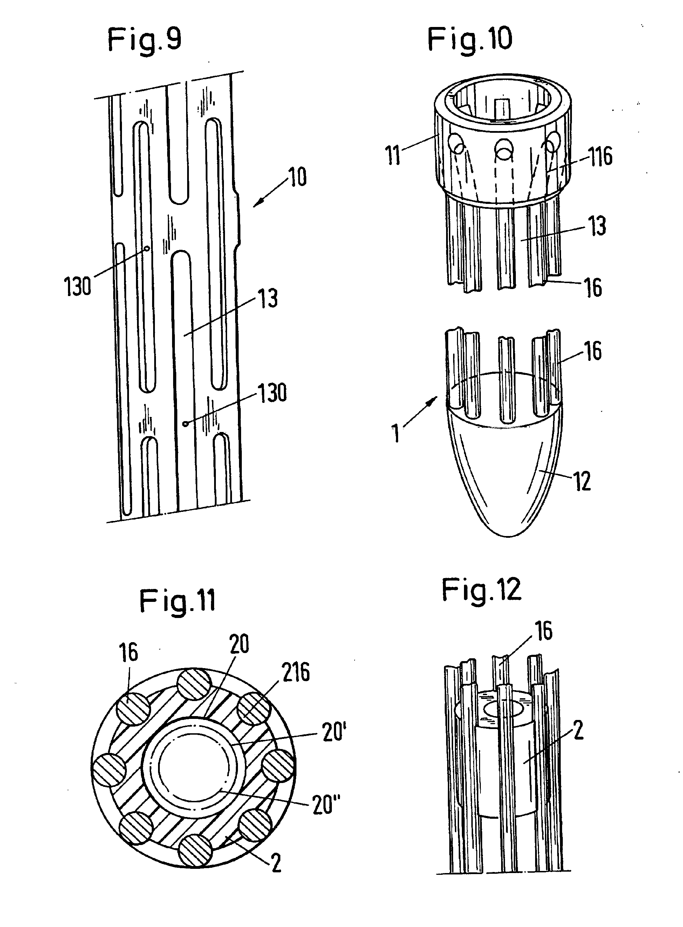

Am proximalen Ende des Rohrs 10, im Mündungsbereich 11, verjüngt sich der Rohrinnenraum in Einschieberichtung. Der proximale Teil ist soweit aufgeweitet, dass die Verstärkungselemente 2 ohne Schwierigkeiten einschiebbar sind. Am distalen Ende ist das Rohr 10 offen. Es kann auch eine geschlossene Spitze aufweisen (vgl. Fig. 10). Das offene Rohrende ist konisch verjüngt, damit sich der Spitzenbereich 12 in der Markhöhle zentriert.

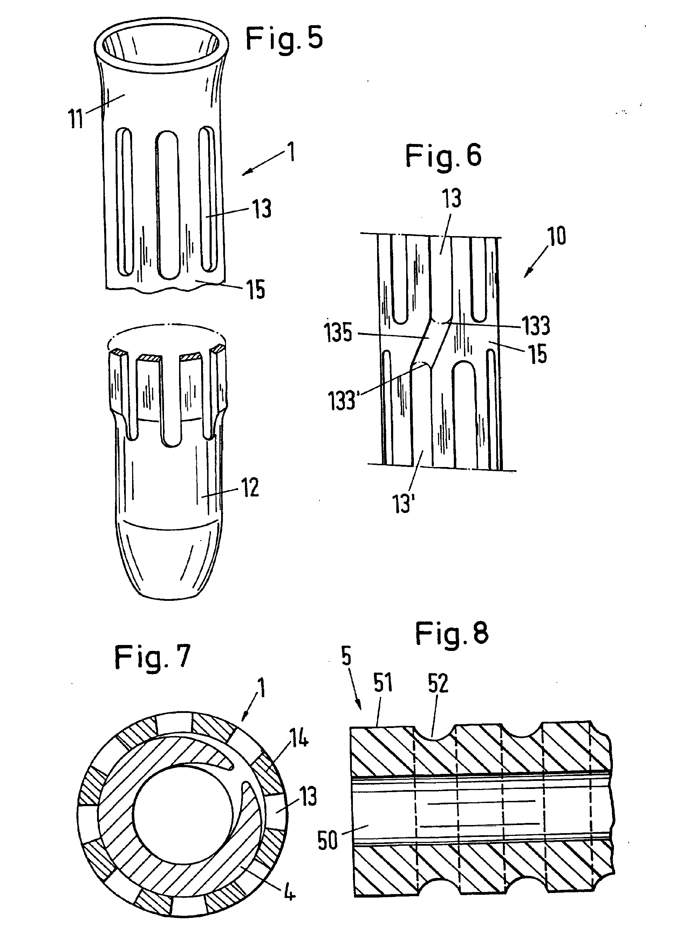

Bei der Ausführungsform der Fig. 5 sind alle Durchbrüche 13 gleich lang und gruppenweise jeweils auf gleicher Höhe über den Umfang des Rohrs 10 verteilt angeordnet. Zwischen diesen Gruppen von Durchbrüchen 13 weist das Rohr 10 geschlossene Ringbereiche 15 auf, die beim Einschieben in den Knochen aufgrund ihrer Starrheit hinderlich sind. Abhilfe - siehe Fig. 6 - kann mittels eines Verbindungsschlitzes 135 geschaffen werden, welcher die Enden 133 und 133' zweier Durchbrüche 13 bzw. 13' benachbarter Gruppen verbindet. Durch den Verbindungsschlitz 135 wird der Ringbereich 15 elastisch nachgiebig. Damit der biegeweiche Bereich des Rohrs 10 über seine ganze Länge elastisch nachgiebig ist, muss ganz allgemein folgendes gelten: Die Durchbrüche müssen so geformt und angeordnet sein, dass in jedem Querschnitt mindestens ein Durchbruch die Rohrwand unterbricht.

Die einschiebbaren Elemente 2 können einzelne Teile sein oder können miteinander zu einem zusammenhängenden Versteifungskörpers verbunden sein. Fig. 7 zeigt einen Querschnitt durch einen erfindungsgemässen Marknagel 1 mit einem Versteifungskörper 4 im Bereich der Durchbrüche 13, welcher in Form eines schraubenförmig gewickelten Drahts ausgebildet ist. Der Draht 4 kann aus einem metallischen Material hergestellt sein; er kann auch aus Kunststoff bestehen oder ein mit Kunststoff beschichteter Draht sein.

Fig. 8 zeigt einen rohrförmigen Versteifungskörper 5, mit einer axialen Bohrung 50, Segmenten 51 und ringförmigen Nuten 52. Die Segmente 51 bilden die versteifenden Teile, die mit der Innenseite des biegeweichen Rohrs 10 in Kontakt gebracht werden. Statt diskreter Segmente 51 kann auch ein zusammenhängender, helixförmiger Streifen vorgesehen sein.

In Fig. 9 ist ein Ausschnitt des in Fig. 2 gezeigten Marknagels 1 abgebildet. Die Längserstreckungen der Durchbrüche 13 sind wesentlich geringer als die Länge des Rohrs 10. Insbesondere haben alle oder zumindest die meisten Durchbrüche 13 gleiche Längen. Sie sind versetzt angeordnet, so dass die Mittelpunkte 130 benachbarter Durchbrüche jeweils um etwas mehr als die halbe Durchbruchlänge von einander entfernt sind.

Beim Ausführungsbeispiel in Fig. 10 ist der biegeweiche Bereich des Rohrs 10 durch eine Mehrzahl von Stäben 16 oder Drähten gebildet. Diese Stäbe 16 sind in Abständen angeordnet, so dass sich offene Durchbrüche 13 ergeben. Im ringförmig geschlossenen Mündungsbereich 11 sind die Enden der Stäbe 16 in Bohrungen 116 unlösbar eingefügt. Das distale Ende des Marknagels 1 trägt eine geschlossene Spitze 12.

Die Figuren 11 und 12 zeigen ein Verstärkungselement 2, das sich für den Marknagel 1 der Fig. 10 eignet. Das Verstärkungselement 2 ist ein axialer Körper mit Nuten 216 an seiner Oberfläche. Die Nuten 216 verlaufen in Achsrichtung, und die Stäbe 16 passen an deren Innenseite formschlüssig in die Nuten 216. Wie bereits im Beispiel der Fig. 3 weisen das Verstärkungselement 2 sowie weitere Verstärkungselemente 2', 2" zentrale Bohrung 20, 20' bzw. 20" auf.

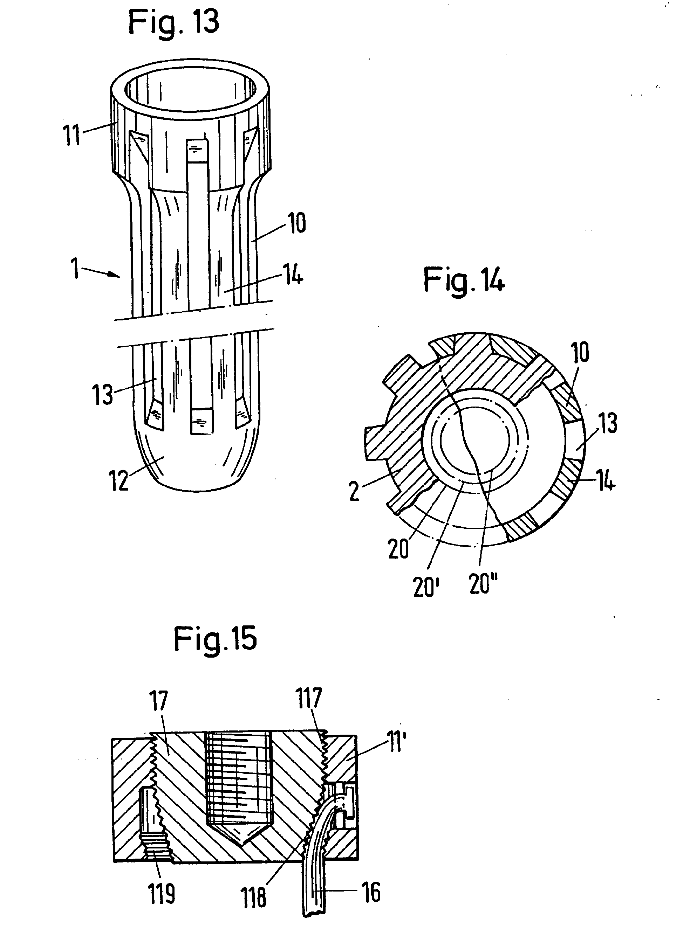

Der Marknagel 1 gemäss Fig. 10 muss aus mehreren Einzelteilen zusammengefügt werden. Das Rohr 10 mit dem Mündungsbereich 11, dem Spitzenbereich 12 und dem biegeweichen, aus Stäben 14 bestehenden Bereich kann auch monolithisch ausgebildet sein. Ein Beispiel ist in Fig. 13 gezeigt. Die Schlitze können gefräst werden; sie können auch mittels Laser oder Wasserstrahl geschnitten werden. Zu diesem Beispiel ist in Fig. 14 ein Querschnitt dargestellt, der entsprechende Verhältnisse wie die Fig. 11 zeigt, wobei das Verstärkungselement 2 und das Rohr 10 als Fragmente dargestellt sind.

Beim Marknagel 1 gemäss Fig. 10 sind die Stäbe 16 im Spitzenbereich 12 und im Mündungsbereich 11 fest verankert. Bei einer weiteren vorteilhaften Ausführungsform sind die Stäbe 16 in einem Mündungsstück 11' mit einem axialen Spiel befestigt: siehe Fig. 15. Nach dem Einschieben der Versteifungselemente 2 in das Rohr 10 werden die Stäbe 16 mit einem Verschlussstück 17 in ihren axialen Lagen fixiert, die sie nach dem Einsetzen des Marknagels 1 einnehmen. Diese axialen Lagen hängen beispielsweise davon ab, ob und wie stark der Marknagel 1 nach dem Einsetzen in den Knochen gebogen ist. Das in Fig. 15 dargestellte Verschlussstück 17 ist in das ringförmige Mündungsstück 11' einschraubbar. Es hat neben einem Gewinde 117 für die Verschraubung einen konischen Bereich mit Rillen 118. Beim Einschrauben graben sich diese Rillen 118 in die nach innen vorstehenden Stäbe 16 ein, die gleichzeitig gegen Rillen 119 des Mündungsstücks 11' gepresst werden.

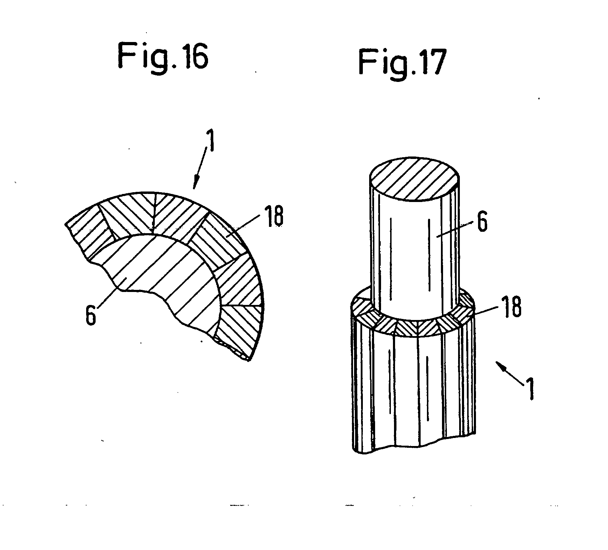

Die Figuren 16 und 17 (Querschnitt in Fig. 16) zeigen Fragmente eines Marknagels 1, dessen rohrfömig angeordneten Stäbe 18 jeweils einen trapezförmigen Querschnitt aufweisen. Der Marknagel 1 wird vorzugsweise aus Stahl gefertigt. Das Verstärkungselement 6 ist ein Stab mit kreisfömigem Querschnitt, der in den durch die Stäbe 18 gebildeten Innenraum passt. Die Steifigkeit des Marknagels wird vor allem durch folgende Grössen bestimmt: radiale Dicke der Stäbe 18, Anzahl der Stäbe 18, Durchmesser des Verstärkungselements 6, E-Modul des für die Stäbe 18 und für das Verstärkungselement 6 verwendeten Werkstoffs.

Bei dieser Ausführungsform wird verhindert, dass der Knochen F in Lücken zwischen den Stäben 18 einwächst und die Extraktion des Marknagels 1 erschwert. An Biegestellen des Marknagels 1 ergeben sich Lücken zwischen den Stäben, in die der Knochen F einwachsen kann. Bei der Extraktion lässt sich der Marknagel 1 verdrehen, so dass dank den scharfkantigen Stäben 18 das eingewachsene Knochenmaterial abgeschert und zerkleinert wird.

## Patentansprüche

1. Marknagel, insbesondere Femurmarknagel (1) für Kinder, ein Rohr (10) und ein in das Rohr einschiebbares Element (2; 4; 5; 6) oder mehrere solche Elemente (2, 2', 2") umfassend, wobei das Rohr ein elastischer Zylinder ist, der sich zwischen einem Mündungsbereich (11) am proximalen Ende und einem Spitzenbereich (12) am distalen Ende erstreckt und der mittels schlitzförmiger, in Rohrrichtung weisender Durchbrüche (13) biegeweich ausgebildet ist, und wobei die eingeschobenen Elemente zumindest lokale Verstärkungen bilden, durch die das Rohr eine über seine Länge global wirkende Versteifung erhält,
**dadurch gekennzeichnet, dass** jeder Durchbruch (13) sowohl in einem axialen Abstand vom proximalen Ende als auch in einem axialen Abstand vom distalen Ende endet, so dass sowohl der Mündungsbereich als auch der Spitzenbereich in Umfangsrichtung geschlossen sind.

2. Marknagel nach Anspruch 1, **dadurch gekennzeichnet, dass** die einschiebbaren Elemente eine Mehrzahl von Körpern (2, 2', 2") umfassen, die einzeln mit einem Montagestab (3) in das Rohr (10) einbringbar sind und die an Verstärkungsstellen aufgrund von Reibkräften zwischen den sich berührenden Oberflächen jeweils einen festen Sitz haben.

3. Marknagel nach Anspruch 1, **dadurch gekennzeichnet, dass** zumindest teilweise die einschiebbaren Elemente ein Teil oder mehrere Teile eines zusammenhängenden Versteifungskörpers (4; 5) bilden, der insbesondere radial steife Bereiche und biegeelastische Verbindungen zwischen diesen Bereichen umfasst.

4. Marknagel nach Anspruch 3, **dadurch gekennzeichnet, dass** Segmente (51) mit ringförmigen Nuten (52) als Zwischenräume die genannten Teile des Versteifungskörpers (5) bilden oder dass ein helixförmiger Streifen mit einem nutförmigen Zwischenraum den genannten Teil des Versteifungskörpers bildet, wobei der helixförmige Streifen auch in Form eines schraubenförmig gewickelten Drahts (4) ausgebildet sein kann.

5. Marknagel nach einem der Ansprüche 1 bis 4, **dadurch gekennzeichnet, dass** das Rohr (10) metallisch ist, beispielsweise aus einem rostfreien Stahl besteht, und die Verstärkungselemente (2; 5; 6) aus Polymeren hergestellt sind, die zumindest zu einem Teil bioresobierbare Polymere sein können.

6. Marknagel nach einem der Ansprüche 1 bis 5, **dadurch gekennzeichnet, dass** der Rohrinnenraum am proximalen Ende (11) sich in Einschieberichtung verjüngt, und dass am distalen Ende (12) das Rohr (10) offen ist oder eine geschlossene Spitze aufweist.

7. Marknagel nach einem der Ansprüche 1 bis 6, **dadurch gekennzeichnet, dass** die Durchbrüche (13) in der Wand des Rohrs (10) so angeordnet sind, dass der Querschnitt an jeder Stelle im biegeweichen Bereich mindestens einen Durchbruch in der Rohrwand aufweist.

8. Marknagel nach einem der Ansprüche 1 bis 7, **dadurch gekennzeichnet, dass** die Längserstreckungen der Durchbrüche (13) wesentlich kleiner als die Länge des Rohrs (10) sind und dass insbesondere alle oder zumindest die meisten Durchbrüche gleiche Längen haben und versetzt angeordnet sind, so dass die Mittelpunkte (130) benachbarter Durchbrüche jeweils um etwas mehr als die halbe Durchbruchlänge von einander entfernt sind.

9. Marknagel nach einem der Ansprüche 1 bis 7, **dadurch gekennzeichnet, dass** der biegeweiche Bereich des Rohrs (10) durch eine Mehrzahl von in Abständen angeordneten Stäben (16) oder Drähten gebildet ist.

10. Marknagel nach Anspruch 9, **dadurch gekennzeichnet, dass** jedes Verstärkungselement (2) ein axialer Körper mit Nuten an seiner Oberfläche ist, wobei die Nuten in Achsrichtung verlaufen und die Stäbe (16) oder Drähte an der Innenseite (216) des biegeweichen Bereichs formschlüssig in die Nuten passen.

11. Marknagel nach Anspruch 9, **dadurch gekennzeichnet, dass** die Stäbe (16) im Mündungsbereich (11) mit axialem Spiel befestigt sind und dass sie nach dem Einsetzen des Marknagels (1) in einen Knochen (F) in ihren momentanen axialen Stellungen im Mündungsbereich fixierbar sind.

12. Marknagel nach einem der Ansprüche 1 bis 7, **dadurch gekennzeichnet, dass** der biegeweiche Bereich des Rohrs durch eine Mehrzahl von dicht angeordneten Stäben (18) gebildet ist, die jeweils einen trapezförmigen Querschnitt aufweisen, wobei vorzugsweise sich benachbarte Stäbe berühren.

## Claims

1. An intramedullary nail, in particular a femoral intramedullary nail (1) for children, comprising a tube (10) and an element (2; 4; 5; 6) which can be pushed into the tube, or a plurality of such elements (2, 2', 2"), wherein the tube is an elastic cylinder which extends between an opening region (11) at the proximal end and a tip region (12) at the distal end and which is formed as flexible by means of slit-like openings (13) facing in the direction of the tube and wherein the pushed in elements form at least local reinforcements by which the tube receives a stiffening acting globally over its length, **characterised in that**
each opening (13) ends both at an axial spacing from the proximal end and at an axial spacing from the distal end so that both the opening region and the tip region are closed in the peripheral direction.

2. An intramedullary nail in accordance with claim 1, **characterised in that** the elements which can be pushed in comprise a plurality of bodies (2, 2', 2") which can be introduced individually into the tube (10) using an installation rod (3) and which each have a fixed seat at reinforcement points due to frictional forces between contacting surfaces.

3. An intramedullary nail in accordance with claim 1, **characterised in that** the elements which can be pushed in at least partly form one part or a plurality of parts of a contiguous stiffening body (4; 5) which in particular comprises radially stiff regions and resiliently elastic connections between these regions.

4. An intramedullary nail in accordance with claim 3, **characterised in that** segments (51) having annular grooves (52) as intermediate spaces form the named parts of the stiffening body (5); or **in that** a helical strip having a groove-shaped intermediate space forms the named part of the stiffening body, with the helical strip also being able to be formed in the form of a helically wound wire (4).

5. An intramedullary nail in accordance with any one of the claims 1 to 4, **characterised in that** the tube (10) is metallic, for example comprises a stainless steel, and the reinforcement elements (2; 5; 6) are manufactured from polymers which can at least partly be bioresorbable polymers.

6. An intramedullary nail in accordance with any one of the claims 1 to 5, **characterised in that** the inner tube space converges in the pushing in direction at the proximal end (11); and **in that** the tube (10) is open or has a closed tip at the distal end (12).

7. An intramedullary nail in accordance with any one of the claims 1 to 6, **characterised in that** the openings (13) are arranged in the wall of the tube (10) such that the cross-section has at least one opening in the tube wall at each point in the flexible region.

8. An intramedullary nail in accordance with any one of the claims 1 to 7, **characterised in that** the longitudinal extents of the openings (13) are substantially smaller than the length of the tube (10); and **in that** in particular all, or at least most, openings have equal lengths and are arranged offset so that the centres (130) of adjacent openings are each remote from one another by a little more than half the opening length.

9. An intramedullary nail in accordance with any one of the claims 1 to 7, **characterised in that** the flexible region of the tube (10) is formed by a plurality of rods (16) or wires arranged at intervals.

10. An intramedullary nail in accordance with claim 9, **characterised in that** each reinforcement element (2) is an axial body having grooves at its surface, with the grooves extending in the axial direction and the rods (16) or wires fitting into the grooves with shape matching at the inner side (216) of the resilient region.

11. An intramedullary nail in accordance with claim 9, **characterised in that** the rods (16) are fastened with axial clearance in the opening region (11); and **in that** they can be fixed in their instantaneous axial positions in the opening region after the insertion of the intramedullary nail (1) into a bone (F).

12. An intramedullary nail in accordance with any one of the claims 1 to 7, **characterised in that** the resilient region of the tube is formed by a plurality of tightly arranged rods (18) which each have a trapezoidal cross-section, with adjacent rods preferably contacting one another.

## Revendications

1. Clou médullaire, en particulier clou médullaire de fémur (1) destiné à des enfants, comprenant un tube (10) et un élément (2 ; 4 ; 5 ; 6) à enfiler dans le tube ou plusieurs éléments (2, 2', 2") de ce type, dans lequel le tube est un cylindre élastique qui s'étend entre une zone d'embouchure (11) à l'extrémité proximale et une zone de pointe (12) à l'extrémité distale, et qui est réalisé souple en flexion au moyen de traversées (13) en forme de fentes s'étendant dans la direction du tube, et dans lequel les éléments enfilés forment au moins des renforts locaux au moyen duquel le tube reçoit une rigidification agissant globalement sur sa longueur,
**caractérisé en ce que**
chaque traversée (13) se termine aussi bien à une distance axiale de l'extrémité proximale qu'à une distance axiale de l'extrémité distale, de sorte que la zone d'embouchure tout comme la zone de pointe sont fermées en direction périphérique.

2. Clou médullaire selon la revendication 1, **caractérisé en ce que** les éléments à enfiler comprennent une pluralité de corps (2, 2', 2"), qui sont susceptibles d'être introduits individuellement avec une tige de montage (3) dans le tube (10) et qui présentent au niveau des emplacements de renforcement respectivement une assise ferme en raison des forces de friction entre les surfaces en contact.

3. Clou médullaire selon la revendication 1, **caractérisé en ce que** les éléments à enfiler forment partiellement une partie ou plusieurs parties d'un corps de rigidification (4 ; 5) d'un seul tenant, qui inclut en particulier des zones radiales rigides et des liaisons élastiques en flexion entre ces zones.

4. Clou médullaire selon la revendication 3, **caractérisé en ce que** des segments (51) avec des gorges de forme annulaire (52) à titre d'espaces intermédiaires forment lesdites parties du corps de rigidification (5), ou en ce qu'un ruban de forme hélicoïdale avec un espace intermédiaire en forme de gorge forme ladite partie du corps de rigidification, et le ruban de forme hélicoïdale peut être également réalisé sous la forme d'un fil (4) enroulé en forme de vis.

5. Clou médullaire selon l'une des revendications 1 à 4, **caractérisé en ce que** le tube (10) est métallique, par exemple réalisé en un acier inoxydable, et les éléments de renforcement (2 ; 5 ; 6) sont réalisés en polymère, qui peuvent être au moins en partie des polymères biorésorbables.

6. Clou médullaire selon l'une des revendications 1 à 5, **caractérisé en ce que** l'espace intérieur du tube va en se rétrécissant en direction d'introduction à l'extrémité proximale (11), et **en ce que** le tube (10) est ouvert à l'extrémité distale (12) ou présente une pointe fermée.

7. Clou médullaire selon l'une des revendications 1 à 6, **caractérisé en ce que** les traversées (13) dans la paroi du tube (10) sont agencées de telle façon que la section transversale présente au moins une traversée dans la paroi du tube à chaque emplacement dans la zone souple en flexion.

8. Clou médullaire selon l'une des revendications 1 à 7, **caractérisé en ce que** les extensions longitudinales des traversées (13) sont sensiblement plus petites que la longueur du tube (10), et **en ce qu'**en particulier toutes les traversées ou tout au moins la plupart des traversées ont la même longueur et sont agencées en décalage de telle façon que les centres (130) de traversées voisines sont respectivement éloignés les uns des autres d'environ plus de la moitié de la longueur des traversées.

9. Clou médullaire selon l'une des revendications 1 à 7, **caractérisé en ce que** la zone souple en flexion du tube (10) est formée par une pluralité de barreaux (16) ou de fils agencés à distance.

10. Clou médullaire selon la revendication 9, **caractérisé en ce que** chaque élément de renforcement (2) est un corps axial avec des gorges à sa surface, dans lequel les gorges s'étendent en direction axiale et les barreaux (16) ou les fils au niveau de la face intérieure (216) de la zone souple en flexion sont ajustés en coopération de formes dans les gorges.

11. Clou médullaire selon la revendication 9, **caractérisé en ce que** les barreaux (16) sont fixés avec jeu axial dans la zone d'embouchure (11), et **en ce qu'**après la mise en place du clou médullaire (1) dans un os (F) ils peuvent être fixés à leurs emplacements axiaux momentanés dans la zone d'embouchure.

12. Clou médullaire selon l'une des revendications 1 à 7, **caractérisé en ce que** la zone souple en flexion du tube est formée par une pluralité de barreaux (18) agencés très proches, qui présentent chacun une section transversale de forme trapézoïdale, et de sorte que de préférence des barreaux voisins se touchent.
